Europäisches Patentamt

(19) European Patent Office

Office européen des brevets

(11) Publication number: **0 193 349 B1**

# EUROPEAN PATENT SPECIFICATION

(45) Date of publication of patent specification: **05.02.92**  (51) Int. Cl.⁵: **C07K 13/00,** C07K 15/00, C07K 3/02, C07K 3/12

(21) Application number: **86301177.1**

(22) Date of filing: **19.02.86**

(54) Isolation, purification and sequence determination of cardionatrins III and IV.

(30) Priority: **27.02.85 US 706379**

(43) Date of publication of application:
**03.09.86 Bulletin 86/36**

(45) Publication of the grant of the patent:
**05.02.92 Bulletin 92/06**

(84) Designated Contracting States:
**BE CH DE FR GB IT LI NL SE**

(56) References cited:
**EP-A- 0 116 784**
**EP-A- 0 166 585**

**BIOCHEMICAL AND BIOPHYSICAL RE-SEARCH COMMUNICATIONS, vol. 121, no. 2, June 15, 1984, pages 585-591; Academic Press Inc. K. KANGAWA et al.: "Identification in rat atrial tissue of multiple forms of natriuretic polypeptides of about 3,000 daltons."**

**NATURE, vol. 309, no. 5970, June 21, 1984, pages 722-724; MacMillan Publ., London, GB M. MAKI et al.: "Structure of rat atrial natriuretic facotr precursor deduced from**

**cDNA sequence."**

(73) Proprietor: **OUEEN'S UNIVERSITY AT KINGS-TON**

**Kingston Ontario K7L 3N6(CA)**

(72) Inventor: **de Bold, Adolfo J.**
**981 Alpine Court**
**Kingston Ontario(CA)**

(74) Representative: **Wood, Anthony Charles et al Urquhart-Dykes & Lord 91 Wimpole Street London W1M 8AH(GB)**

**Description**

This invention relates to peptides which may be isolated from mammalian heart atria and which exhibit potent diuretic, natriuretic, vasodilatory and cardioinhibitory properties. They are also inhibitors of production and/or release of aldosterone and arginine vasopressin.

It has been known for several years that the muscle cells of the atrial myocardium in mammals contain, in addition to contractile elements similar to those found in ventricular fibers, a highly developed Golgi complex, a relatively high proportion of rough endoplasmic reticulum, and numerous membrane bound storage granules, referred to as specific atrial granules. No such granules appear to exist in ventricular muscle cells. Peptide isolates from the atrial granules have been shown to exhibit potent diuretic, natriuretic and vasodilatory properties. In our earlier application 83307877.7 (EP-A-0116784) filed December 22nd 1983 a peptide defined by 28 amino acid residues was described and that peptide was defined as a 28 residue disulfide looped structure and named Cardionatrin I. In applicatation 85107916.0 (EP-A-0172361) filed June 26th 1985, the sequence of a cloned cDNA for rat cardionatrin precursor has been established. The precursor, preprocardionatrin, has been shown to contain 152 amino acids and cardionatrin I containing a 28 amino acid sequence beginning at residue 123 thereof may be cleaved therefrom.

Other have verified the preprocardionatrin sequence and have reported a multitude of different peptides which differ from Cardionatrin I by a few amino acids more or less. In contrast, I have consistently observed only three other bio active peptides, cardionatrins II, III and IV (as hereinafter defined) which have significantly large molecular weights than Cardionatrin I, and which can be derived, from a common precursor.

EP-A-0166585 falling within the state of the art according to article 54(3)EPC discloses a DNA fragment comprising a base sequence coding for a peptide occuring in rat atrium cordis and having diuretic action, or for a precursor of the peptide. It also discloses a peptide having an amino acid composition

| Asn | Pro | Val | Tyr | Ser | Ala | Val | Ser | Asn | Thr | Asp |
| Leu | Met | Asp | Phe | Lys | Asn | Leu | Leu | Asp | His | Leu |
| Glu | Glu | Lys | Met | Pro | Val | Glu | Asp | Glu | Val | Met |
| Pro | Pro | Gln | Ala | Leu | Ser | Glu | Gln | Thr | Asp | Glu |
| Ala | Gly | Ala | Ala | Leu | Ser | Ser | Leu | Ser | Glu | Val |
| Pro | Pro | Trp | Thr | Gly | Glu | Val | Asn | Pro | Ser | Gln |
| Arg | Asp | Gly | Gly | Ala | Leu | Gly | Arg | Gly | Pro | Trp |
| Asp | Pro | Ser | Asp | Arg | Ser | Ala | Leu | Leu | Lys | Ser |
| Lys | Leu | Arg | Ala | Leu | Leu | Ala | Gly | Pro | Arg | Ser |
| Leu | Arg | Arg | Ser | Ser | Cys | Phe | Gly | Gly | Arg | Ile |
| Asp | Arg | Ile | Gly | Ala | Gln | Ser | Gly | Leu | Gly | Cys |
| Asn | Ser | Phe | Arg | Tyr. |

optionally terminated by up to two arginine residues. The peptide without the terminal arginine residues appears to be identical with the material hereinafter identified as cardionatrin IV.

In satisfaction of the objects of this invention there is provided a process for the preparation of a purified bioactive peptide (Cardionatrin III) having an amino acid sequence

2

| Leu | Ser | Ser | Leu | Ser | Glu | Val | Pro | Pro | Trp | Thr |
|-----|-----|-----|-----|-----|-----|-----|-----|-----|-----|-----|
| Gly | Glu | Val | Asn | Pro | Ser | Gln | Arg, | Asp | Gly | Gly |
| Ala | Leu | Gly | Arg | Gly | Pro | Trp | Asp | Pro | Ser | Asp |
| Arg | Ser | Ala | Leu | Leu | Lys | Ser | Lys | Leu | Arg | Ala |
| Leu | Leu | Ala | Gly | Pro | Arg | Ser | Leu | Arg | Arg | Ser |
| Ser | Cys | Phe | Gly | Gly | Arg | Ile | Asp | Arg | Ile | Gly |
| Ala | Gln | Ser | Gly | Leu | Gly | Cys | Asn | Ser | Phe | Arg | Tyr |

which comprises

(a) extracting said Cardionatrin III from rat atria using a strongly acid medium; and

(b) purifying said bioactive peptide (Cardionatrin III) composition by adsorption on octadecylsilylsilica, and a combination of high and low pressure liquid chromatography.

In still further satisfaction there is provided a purified bioactive composition containing a peptide having a molecular weight of about 8,000 and having an amino acid sequence

| | | | | | | | | | | Leu, |
|-----|-----|-----|-----|-----|-----|-----|-----|-----|-----|-----|
| Ser | Ser | Leu | Ser | Glu | Val | Pro | Pro | Trp | Thr | Gly |
| Glu | Val | Asn | Pro | Ser | Gln | Arg | Asp | Gly | Gly | Ala |
| Leu | Gly | Arg | Gly | Pro | Trp | Asp | Pro | Ser | Asp | Arg |
| Ser | Ala | Leu | Leu | Lys | Ser | Lys | Leu | Arg | Ala | Leu |
| Leu | Ala | Gly | Pro | Arg | Ser | Leu | Arg | Arg | Ser | Ser |
| Cys | Phe | Gly | Gly | Arg | Ile | Asp | Arg | Ile | Gly | Ala |
| Gln | Ser | Gly | Leu | Gly | Cys | Asn | Ser | Phe | Arg | Tyr. |

Fig.1a is a graph illustrating gel permeation chromatography of atrial extracts

Figs.1b- 1e are graphs illustrating purification of Cardionatrins I,II,III & IV respectively by HPLC.

Fig. 2 is a graph illustrating SDS-urea-polyacrylamide gel electrophoresis of Cardionatrin IV

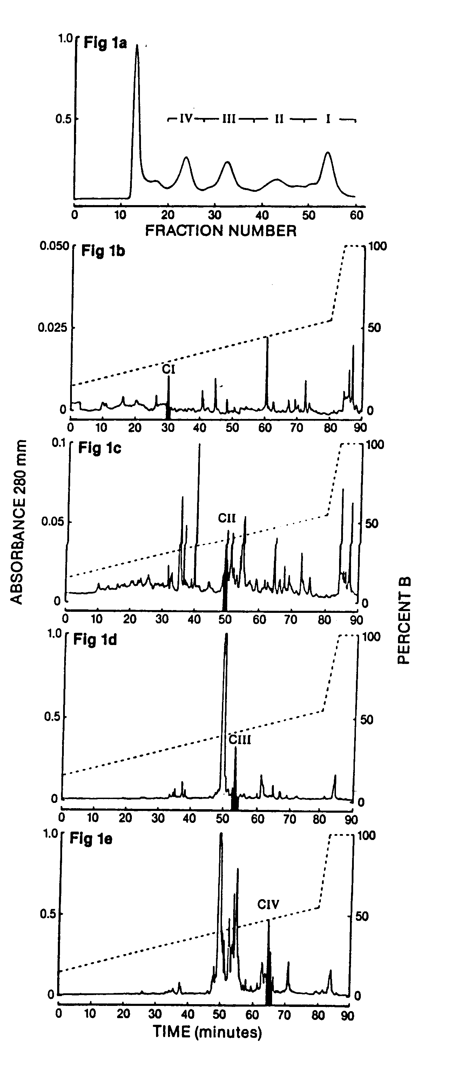

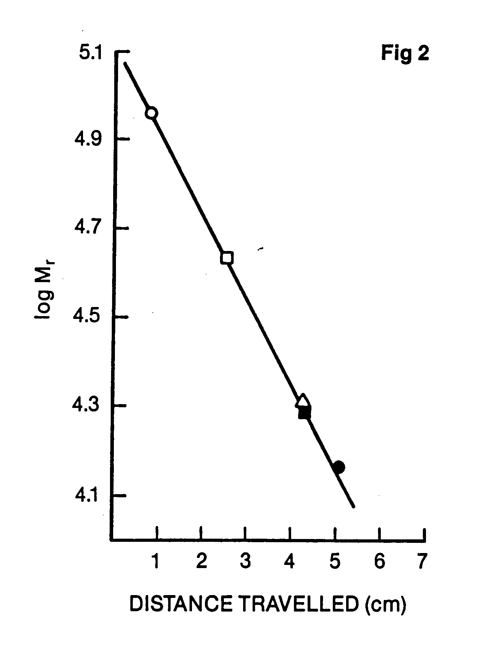

For the purposes of this application it has been established previously that all mammalian-heart atria tested contain the specific granules referred to above and that neither the diet of the mammal nor the physical condition i.e. fresh, fresh-frozen or frozen, of the atria has any significant effect upon the extracted product. The peptide extract yield from different species may vary considerably but the composition of the extract for each species is remarkably consistent. There may be minor differences in amino acid composition between extracts from different species. Thus, as rat atria have been shown to contain a relatively high concentration of the cardionatric peptides (relative to beef, human and other mammalian atria), one thousand frozen rat atria from non specific commercial sources were employed. The atria were ground together with solid carbon dioxide chips and homogenized in 10 volumes of a strongly acidic medium comprising (w/v) 1.0 M acetic acid/l.0N HCl/1% NaCl using a Polytron ®. After centrifugation of the extracts, 20 ml aliquots of supernatants were passed through pre-wetted $C_{18}$ Sep-Pak ® (Waters) cartridges which contain octadecylsylilsilica. Each cartridge was washed with 20 ml of 0.1% trifluoracetic acid (TFA) and eluted with 3 ml of 80% acetonitrile in 0.1% TFA. The combined cartridge eluates were freeze-dried, then dissolved in 5 ml of 1.0 M acetic acid/1% NaCl and fractionated on a Bio-Gel ® P-10 column (2.5 x 40 cm) equilibrated with the same solution. Fraction pools I,II,III and IV indicated in Fig. 1a were further processed by high pressure liquid chromatography (HPLC) by directly pumping them into two serially connected Vydac ® C$_{18}$ columns (10 x 250 mm) preequilibrated with 12% acetonitrile in 0.1% TFA. Elution was carried out at 3.0 ml/min with acetonitrile gradients (12-44%) containing 0.1% TFA. These gradients were achieved by varying the input of the "organic" pump (%B, Figs. 1b-e) which delivered an 80% (v/v) acetonitrile solution in 0.1% TFA. Elution times for cardionatrins I,II, III and IV were determined by the rat bioassay and are indicated in 1b, 1c, 1d, 1e, as CI, CII, CIII and CIV respectively. Cardionatrins I and II were isolated as described in Biochem-Biophys Res. Comm 117, 859-865 (1983), and further purification in similar acetonitrile gradients but containing 0.13% heptafluorobutyric acid as counter ion. Cardionatrins III and IV were further purified by high performance cation exchange chromatography (Spherogel ® TSK, 4 x 300 mm, Altex) using a gradient over 60 minutes at 1.0 ml/min of 0.010 M to 1.0 M ammonium formate buffer, pH 6.5, containing 10% acetonitrile throughout. All cardionatrins went through a final purification step performed in a Vydac ® C$_{18}$ column (4.6 x 250 mm) eluted at 1.5 ml/min with acetonitrile gradients containing 0.1% TFA. SDS-urea-polyacrylamide gel electrophoresis for cardionatrin IV was performed using essentially the technique described by Laemmli in Nature 227, 680-685 (1970), as shown in Fig. 2. The molecular weights of standard proteins were a) 92,500 (phosphorylase B); b) 66,200 (bovine serum albumin; c) 45,000 (ovalbumin; d) 31,00 (carbonic anhydrase); e) 21,500 (soybean trypsin inhibitor) and f) lysozyme (14,300). The standard curve was constructed using ovalbumin (O), carbonic anhydrase (□), soybean trypsin inhibitor (Δ) and lysozyme (●). Cardionatrin IV is indicated by (■).

## PREPROCARDIONATRIN AND ITS CLEAVAGE PRODUCTS

Poly (A) + RNA was isolated from rat atria as previously described in co-pending U.S. Patent application 626,219 and used to prepare cDNA clones. In this instance the double-stranded cDNAs were G-tailed and annealed into the C-tailed Pst I site of pUC 9 which was then used to transform E.coli JM83 to ampicillin resistance. Colonies (650) which remained white in the presence of X-gal were screened for hybridization to the nick-translated P-labelled insert from the cardionatrin cDNA clone car 3. DNA from one of the eleven clones that hybridized (car 60) was end-labelled using the Klenow fragment of E.coli DNA polymerase I and sequenced from the Bgl II sites at 166 bp and 379 bp, the AWva Ii site at 367 bp, the Acc I site at 143 bp, the Xho I site at 536 bp, the Cla I site at 657 bp, and from the Hind III and Acc I sites in the multiple cloning site of pUC 9 flanking the cDNA insert. Two putative poly-adenylation signals are underlined in Fig. 3. The n-terminal sequences of cardionatrins IV and III are indicated by arrows aligned to the preprocardionatrin sequence. These sequences were determined by automatic Edman degradation using an Applied Biosystems Gas-Phase 470A Sequencer and analyzing the phenylthiohydantoin amino acids.

Cardionatrin I was indentical to that isolated and sequenced previously as described in EP-A-116784. Cardionatrin IV had a molecular weight of between 13,600 and 13,700 and more accurately 13,663 according to urea-SDS-polyacrylamide gel electrophoresis (Fig. 2). The primary structure of the first 30 residues of this peptide was determined by stepwise Edman degradation of the unmodified protein using a gas phase sequencer (Applied Biosystems, model 470A). The sequence was identical with residues 25-54 of the anticipated preproprotein sequence derived from a cloned cDNA sequence Both the molecular weight and NH$_2$-terminal sequence of cardionatrin IV are consistent with it being procardionatrin, but the amino acid composition (Table 1 below) suggests that it lacks the two COOH-terminal arginine residues.

Table 1

| Amino acid compositions of cardionatrins I, III and IV | | | |
|---|---|---|---|
| Amino Acid | CI | CIII | CIV |
| Asx | 2.19 (2) | 5.9 (6) | 13:3 (14) |
| Thr | | 0.97 (1) | 3.5 (3) |
| Ser | 4.72 (5) | 11.6 (12) | 13.4 (15) |
| Glx | 1.39 (1) | 4.8 (4) | 11.3 (12) |
| Pro | | 6.2 (6) | 9.6 (10) |
| Gly | 5.03 (5) | 10.7 (11) | 13.1 (12) |
| Ala | 1.32 (1) | 5.2 (5) | 8.7 (10) |
| 1/2Cys | 2.40 (2) | 2.3 (2) | 1.8 (2) |
| Val | | 2.2 (2) | 5.9 (6) |
| Met | | | 1.7 (3) |
| Ile | 2.37 (2) | 2.4 (2) | 2.2 (2) |
| Leu | 2.16 (2) | 9.4 (2) | 15.2 (15) |
| Tyr | 1.37 (1) | 0.8 (1) | 2.6 (2) |
| Phe | 2.28 (2) | 2.8 (2) | 3.8 (3) |
| Lys | | 1.7 (2) | 3.1 (4) |
| His | | | 0.7 (1) |
| Arg | 4.73 (5) | 9.5 (10) | 8.4 (10) |
| Total residues | 28 | 76 | 124 |

Results of a 24 hr hydrolysis in constant boiling 6NHCl and analysis on a Beckman 119c amino acid analyzer.

Values in parenthesis indicate residues found in sequence.

Totals for CIII and CIV do not include Trp.

The amino terminal sequence of cardionatrin IV beginning with asparagine is the predicted cleavage point for the removal of the signal sequence. The analogous position in the human natriuretic factor precursor was shown to be a cleavage point by the isolation and characterization of - ANP, which is the human analog of cardionatrin IV. A further point of interest is that NH - terminus of cardionatrin IV is in register with, and matches almost exactly, the NH -terminal sequence of cardiodilatin, a peptide with smooth muscle relaxant activity isolated from porcine atria. The two differences in sequence - Gly-Ser in positions 5 and 6 of cardiodilatin compared to Ser-Ala at the same positions in rat procardionatrin - reflect the conserved nature of this peptide in different species.

Both cardionatrin II and III are present in such small amounts in acid extracts of rat atria that is has not been possible to characterize them extensively. However, amino acid sequence determination of the first seventeen residues of cardionatrin III located the amino terminus of this molecule at residue 73 of the preproprotein. Cardionatrin III has a calculated molecular weight between 8,000 and 8,500 or more accurately about 8,325. The small amounts of purified cardionatrin II thus far recovered from atrial extracts (approx. 100pmol per 1000 atria) has precluded accurate sequencing of this peptide. Since all four cardionatrins are natriuretic it is expected that they contain most of the sequence of cardionatrin I. Compositional analysis of cardionatrins III and IV (Table I above) indicated that these proteins extend up to and include tyrosine 150.

Identification of the start points for cardionatrin I and III in procardionatrin confirms that these products are derived from a common precursor, cardionatrin IV.

In independently establishing the nucleotide sequence of a cardionatrin complementary DNA clone 650 atrial cDNA clones were screened with a 250 bp cardionatrin cDNA probe. Eleven clones hybridized to the probe. Their cDNA inserts were released by digestion with Bam HI and Eco RI and compared for size. Three inserts were close to full-length, based on previous estimates for the size of cardionatrin mRNA and, one of these, car 60, was sequenced. the cDNA sequence, excluding the poly(a) tract and homopolymeric tracts, is 787 bp long, including 456 bp coding for the 152 residue cardionatrin preproprotein, and 64 bp and 267 bp for the 5' and 3' untranslated regions, respectively.

**Claims**

1. A process for the preparation of a purified bioactive peptide having an amino acid sequence

```
                                              Leu  Ser
Ser  Leu  Ser  Glu  Val  Pro  Pro  Trp  Thr  Gly  Glu
Val  Asn  Pro  Ser  Gln  Arg  Asp  Gly  Gly  Ala Leu  Gly
Arg  Gly  Pro  Trp  Asp  Pro  Ser  Asp  Arg  Ser  Ala
Leu  Leu  Lys  Ser  Lys  Leu  Arg  Ala  Leu  Leu  Ala
Gly  Pro  Arg  Ser  Leu  Arg  Arg  Ser  Ser  Cys  Phe
Gly  Gly  Arg  Ile  Asp  Arg  Ile  Gly  Ala, Gln  Ser
Gly  Leu  Gly  Cys  Asn  Ser  Phe  Arg  Tyr
```

which comprises:
(a) extracting said peptide from rat atria using a strongly acid medium and
(b) purifying said bioactive peptide by adsorption on octadecysylilsilica, and a combination of low and high pressure liquid chromatography.

2. A process as claimed in claim 1 wherein said purified bioactive peptide is further purified by high performance cation exchange chromatography.

3. A purified bioactive composition containing a peptide having a molecular weight of about 8,000 and having an amino acid sequence

```
                              Leu  Ser  Ser  Leu  Ser
Glu  Val  Pro  Pro  Trp  Thr  Gly  Glu  Val  Asn  Pro
Ser  Gln  Arg  Asp  Gly  Gly  Ala  Leu  Gly  Arg  Gly
Pro  Trp  Asp  Pro  Ser  Asp  Arg  Ser  Ala  Leu  Leu
Lys  Ser  Lys  Leu  Arg  Ala  Leu  Leu  Ala  Gly  Pro
Arg  Ser  Leu  Arg  Arg  Ser  Ser  Cys  Phe  Gly  Gly
Arg  Ile  Asp  Arg  Ile  Gly  Ala  Gln  Ser  Gly  Leu
Gly  Cys  Asn  Ser  Phe  Arg  Tyr.
```

**Revendications**

1. Procédé de préparation d'un peptide bioactif purifié ayant la séquence d'acides aminés

```
                                                                    Leu   Ser
        Ser   Leu   Ser   Glu   Val   Pro   Pro   Trp   Thr   Gly   Glu
        Val   Asn   Pro   Ser   Gln   Arg   Asp Gly Gly Ala Leu Gly
        Arg   Gly   Pro   Trp   Asp   Pro   Ser   Asp   Arg   Ser   Ala
        Leu   Leu   Lys   Ser   Lys   Leu   Arg   Ala   Leu   Leu   Ala
        Gly   Pro   Arg   Ser   Leu   Arg   Arg   Ser   Ser   Cys   Phe
        Gly   Gly   Arg   Ile   Asp   Arg   Ile   Gly   Ala   Gln   Ser
        Gly   Leu   Gly   Cys   Asn   Ser   Phe   Arg   Tyr
```

qui comprend :
    (a) l'extraction dudit peptide des oreillettes de rat en utilisant un milieu fortement acide et
    (b) la purification dudit peptide bioactif par adsorption sur de l'octadécylsilylsilice ; et une combinaison de chromatographie en phase liquide à basse et haute pression.

2. Procédé selon la revendication 1, où ledit peptide bioactif purifié est encore purifié par chromatographie par échange de cations de haute performance.

3. Composition bioactive purifiée contenant un peptide ayant un poids moléculaire d'environ 8.000 et ayant une séquence d'acides aminés

```
                                                    Leu Ser Ser Leu Ser
        Glu   Val   Pro   Pro   Trp   Thr   Gly   Glu   Val   Asn   Pro
        Ser   Gln   Arg   Asp   Gly   Gly   Ala   Leu   Gly   Arg   Gly
        Pro   Trp   Asp   Pro   Ser   Asp   Arg   Ser   Ala   Leu   Leu
        Lys   Ser   Lys   Leu   Arg   Ala   Leu   Leu   Ala   Gly   Pro
        Arg   Ser   Leu   Arg   Arg   Ser   Ser   Cys   Phe   Gly   Gly
        Arg   Ile   Asp   Arg   Ile   Gly   Ala   Gln   Ser   Gly   Leu
        Gly   Cys   Asn   Ser   Phe   Arg   Tyr.
```

**Patentansprüche**

1. Verfahren zur Herstellung eines gereinigten bioaktiven Peptids mit der folgenden Aminosäuresequenz:

```
                                           Leu   Ser   Ser   Leu   Ser
    Glu   Val   Pro   Pro   Trp   Thr   Gly   Glu   Val   Asn   Pro
    Ser   Gln   Arg   Asp   Gly   Gly   Ala   Leu   Gly   Arg   Gly
    Pro   Trp   Asp   Pro   Ser   Asp   Arg   Ser   Ala   Leu   Leu
    Lys   Ser   Lys   Leu   Arg   Ala   Leu   Leu   Ala   Gly   Pro
    Arg   Ser   Leu   Arg   Arg   Ser   Ser   Cys   Phe   Gly   Gly
    Arg   Ile   Asp   Arg   Ile   Gly   Ala   Gln   Ser   Gly   Leu
    Gly   Cys   Asn   Ser   Phe   Arg   Tyr.
```

bei dem man

    (a) das Peptid aus Ratten-Herz-Vorkammern mit einem stark sauren Medium extrahiert und
    (b) das bioaktive Peptid durch Adsorption an Octadecylsilylsiliciumdioxid und einer Kombination von Nieder- und Hochdruckflüssig-Chromatographie reinigt.

**2.** Verfahren nach Anspruch 1, bei dem man das gereinigte bioaktive Peptid ferner durch Hochleistungskationen-Austausch-Chromatographie reinigt.

**3.** Gereinigte bioaktive Zusammensetzung, enthaltend ein Peptid mit einem Molekulargewicht von etwa 8.000 und mit der folgenden Aminosäuresequenz:

```
                                           Leu   Ser   Ser   Leu   Ser
    Glu   Val   Pro   Pro   Trp   Thr   Gly   Glu   Val   Asn   Pro
    Ser   Gln   Arg   Asp   Gly   Gly   Ala   Leu   Gly   Arg   Gly
    Pro   Trp   Asp   Pro   Ser   Asp   Arg   Ser   Ala   Leu   Leu
    Lys   Ser   Lys   Leu   Arg   Ala   Leu   Leu   Ala   Gly   Pro
    Arg   Ser   Leu   Arg   Arg   Ser   Ser   Cys   Phe   Gly   Gly
    Arg   Ile   Asp   Arg   Ile   Gly   Ala   Gln   Ser   Gly   Leu
    Gly   Cys   Asn   Ser   Phe   Arg   Tyr.
```

Fig 1a

Fig 1b

Fig 1c

Fig 1d

Fig 1e

Fig 2